# EUROPEAN PATENT SPECIFICATION

(11) **EP 3 494 942 B1**
(45) Date of publication and mention of the grant of the patent: **26.08.2020**
(21) Application number: 18209871.5
(22) Date of filing: 03.12.2018
(51) Int. Cl.: A61F 13/49, A61F 13/494, A61F 13/496, A61F 13/53

(54) **DISPOSABLE WEARABLE ARTICLE**
WEARABLE-EINWEGARTIKEL
ARTICLE VESTIMENTAIRE JETABLE

(30) Priority: 05.12.2017 JP 2017233530
(43) Date of publication of application: 12.06.2019
(73) Proprietor: Unicharm Corporation, Shikokuchuo-shi, Ehime 799-0111 (JP)
(72) Inventor: Kawakami, Yusuke, Kanonji-shi, Kagawa 769-1602 (JP); Gao, Juyi, Kanonji-shi, Kagawa 769-1602 (JP)
(74) Representative: Dolleymores

(56) References cited:
- EP-A1- 1 199 059
- EP-A1- 2 087 871
- EP-A1- 2 275 065
- WO-A1-2006/068549

## Description

### [Technical Field]

The present disclosure relates to a disposable wearable article, and more particularly, to a disposable diaper, disposable toilet-training pants, and disposable incontinence pants.

### [Background]

Conventionally, disposable wearable articles are known having a vertical direction and a lateral direction, and including a skin-facing surface, a skin non-facing surface, a front waist region, a rear waist region, a crotch region positioned between the front and rear waist regions, and a liquid absorbing structure having an absorbent core, extending from the crotch region to the front and rear waist regions.

For example, Patent Literature 1 discloses a disposable diaper including an absorbent core having a core piece divided into three in a width direction, in a crotch region, in which an inter-core area with no absorbent core is positioned between the core pieces.

### [Citation List]

### [Patent Literature]

Patent Literature 1: Japanese Patent Application Laid-open Publication No. 2007-260148
EP 2275065 A1 discloses a disposable wearing article constructed so that an absorbent core may be deformed to form absorbent barriers against body waste: the absorbent core is formed with deformation guides so that at least opposite lateral regions of said absorbent core may be deformed along the deformation guides toward the wearer's torso under elastic contraction of at least a front waist panel.

Other documents in this field are EP 1199059 A1, EP 2087871 A1, WO 2006/068549 A1 and US 20130046266.

### [Summary]

### [Technical Problem]

According to the disposable diaper disclosed in Patent Literature 1, the inter-core area with no absorbent core is between the core piece divided into three, and at the time of putting on the crotch wearable, the crotch region is put smoothly in between the thighs of a wearer. This makes it possible to put the diaper on the wearer.

However, in the diaper disclosed in Patent Literature 1, at the time of putting the diaper on the wearer, because the diaper has the inter-core area, a middle part in the width direction of the absorbent core is deformed to be away from the wearer's skin, in other words, deformed in a generally U-shape, deformed such that the middle part in the width direction is positioned downward, and both lateral sides of the absorbent core are positioned above the middle part, and body exudates such as urine may leak from the diaper.

An object of the present invention is to improve the conventional disposable diaper, and to provide a disposable wearable article such as a disposable diaper capable of preventing the body exudates from leaking from the wearable article.

### [Solution to Problem]

The present invention provides the disposable wearable article of independent claim 1. The dependent claims specify preferred but optional features.

To solve the above-mentioned problems, the present invention is directed to a disposable wearable article having a vertical direction and a lateral direction, including a front waist region, a rear waist region, a crotch region positioned between the front and rear waist regions, and an absorbent body positioned at least in the crotch region.

The present invention is featured in that the absorbent body of the disposable wearable article according to the present invention has both lateral sides of the absorbent body opposite to each other in the lateral direction in the crotch region, and laterally spaced deformation guiding portions extending in the vertical direction in both the lateral sides, and a pair of elastic side flaps extending in the vertical direction are positioned at outer sides in the lateral direction of both of the lateral sides of the absorbent body, and a dimension in the lateral direction of each of the elastic side flaps is larger than a dimension in the lateral direction from both lateral side edges of the absorbent body to a lateral outer edge of each of the deformation guiding portions.

The deformation guiding portions are preferably positioned only in the crotch region, and the absorbent body can be prevented from being deformed in the front and rear waist regions. Thus, the appearance of the disposable wearable article in the worn state can be improved.

In the worn state, in one embodiment, each of the deformation guiding portions have upper and lower end edges in the vertical direction wherein the upper end edge positioned on a side of the front waist region is positioned above the lower end edge positioned on a side of the rear waist region, and on the front waist region side of the crotch region, body exudates such as urine can be prevented from leaking by deforming the absorbent body to be in close contact with the skin of the wearer, whereas on the rear waist region side of the crotch region, the deformation of the absorbent body can be prevented, and the buttocks of the wearer can be covered presentably.

The rear waist region may have a pair of elastic areas spaced apart oppositely in the lateral direction, and an inelastic area positioned between the elastic areas, and the deformation guiding portions are positioned between a pair of virtual lines extending along boundary lines of the elastic areas and the inelastic area toward the crotch region. Thus, a caretaker, at the time of putting on the wearable article by holding it by the hands and pulling upward the middle part in the lateral direction of the rear waist region, applies a force to the middle part of the rear waist region and this force is applied between both lateral sides of the absorbent body. As a result of an absorbent core having high rigidity being disposed continuously in the vertical direction, the force pulling the wearable article upward can be prevented from being reduced.

The wearable article further may include a liquid absorbent structure including the absorbent body and an outer surface sheet positioned on the skin non-facing surface side of the absorbent body, wherein the elastic side flaps have a plurality of leg elastic members extending in the vertical direction, and base sheets having the leg elastic members attached thereto, and the base sheets and the outer surface sheet are formed integrally. Thus, the base sheets and the outer surface sheet being directly connected, when the elastic side flaps are brought into close contact with the skin of the wearer by the elasticity of the elastic side flaps, both lateral sides of the absorbent body can be brought into close contact with the wearer's skin, and thus the body exudates can be prevented from leaking.

The liquid absorbent structure further may include a liquid permeable leakage barrier sheet interposed between the absorbent body and the outer surface sheet, and the leakage barrier sheet extends from the both lateral edges of the absorbent body to further outward in the lateral direction, and a dimension in the lateral direction of the leakage barrier sheet positioned on each of the elastic side flaps is not less than 50% of the dimension in the lateral direction of each of the elastic side flaps. Thus, the absorbent body and the elastic side flaps being connected by the leakage barrier sheet, when the elastic side flaps are brought into close contact with the wearer's skin by the elasticity of the elastic side flap, both lateral sides of the absorbent body can be brought into close contact with the wearer's skin. Thus, the body exudates can be prevented from leaking from the wearable article.

An interval of the leg elastic members may become narrower gradually toward both lateral side edges of the liquid absorbent structure. Thus, when the elastic side flaps are brought into close contact with the wearer's skin toward both lateral side edges of the liquid absorbent structure, the body exudates can be prevented from leaking from the wearable article.

### [Advantageous Effects of Invention]

In the disposable wearable article according to the present invention, the absorbent body has both lateral sides of the absorbent body opposite to each other in the lateral direction in the crotch region, and deformation guiding portions extending in the vertical direction in both lateral sides, and elastic side flaps extending in the vertical direction are positioned at an outer side in the lateral direction of both lateral sides of the absorbent body, and a dimension in the lateral direction of each of the elastic side flaps is larger than a dimension in the lateral direction from both lateral side edges of the absorbent body to an outer side edge of each of the deformation guiding portions adjacent to both the lateral side edges of the absorbent body. Thus, at the time of putting on the wearable article, while the elastic side flaps are fastened at a position of contact with the thighs of the wearer because of a frictional resistance, the elastic side flaps are deformed to be concave downward with respect to the deformation guiding portions by an effect of the deformation guiding portions, and because the absorbent body is brought into close contact with the skin of the wearer, body exudates such as urine can be prevented from leaking from the wearable article.

### [Brief Description of Drawings]

Fig.1 is a perspective view of a disposable diaper, illustrating an example of a disposable wearable article according to the present invention.
Fig.2 is a partially cutaway development plan view of the diaper extending in a vertical direction and a lateral direction when each elastic member is extended to the maximum length.
Fig. 3 is an exploded perspective view of the diaper.
Fig. 4 is an enlarged plan view of a liquid absorbent structure.

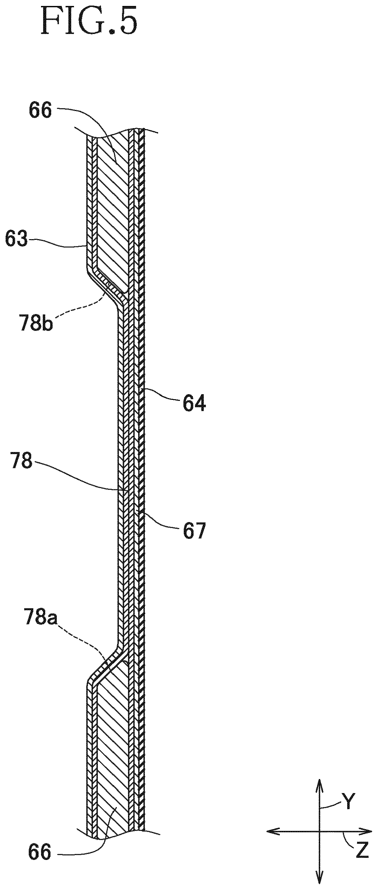

Fig. 5 is a cross-sectional view of arrow V-V in Fig. 4.
Fig. 6 is (a) cross-sectional view illustrating an action of an absorbent body and elastic side flaps initially at the time of putting on the diaper, and (b) cross-sectional view illustrating an effect of the absorbent body and the elastic side flaps at the time of putting on the diaper.
Fig. 7 is a plan view similar to Fig. 4, of a diaper of a first modified embodiment. Fig. 8 is a plan view similar to Fig. 4, of a diaper of a second modified embodiment.

### [Description of Embodiments]

The embodiments described below relate to diapers as illustrated in the accompanying diagrams, including both optional and preferred features as well as those features which are essential to the invention.

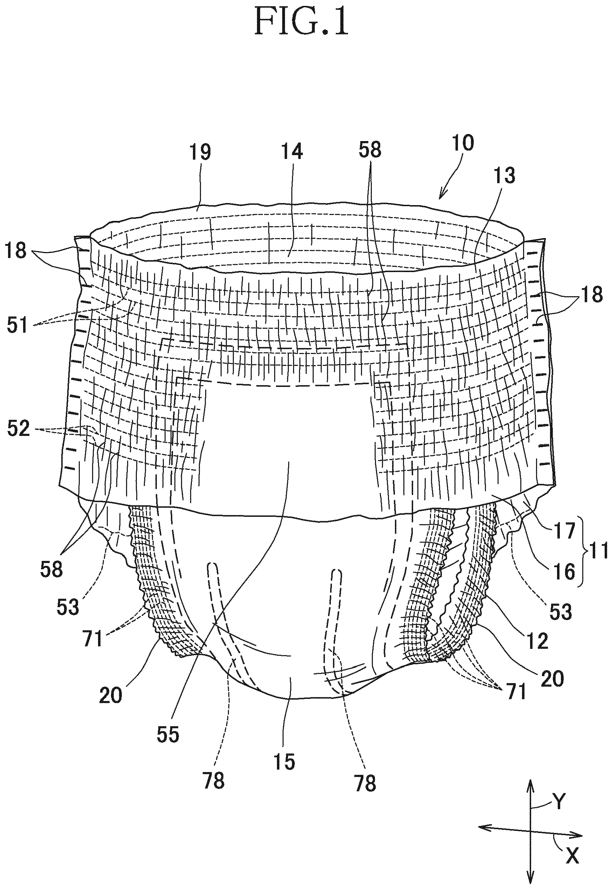

Referring to Fig. 1 to Fig. 3, a disposable diaper 10 that is an example of a disposable wearable article according to the present invention has a lateral direction X and a vertical direction Y orthogonal to each other, and a skin facing surface and a skin non-facing surface opposite thereto, and may include a waist panel 11 having elasticity and a liquid absorbent structure 12 attached to the waist panel 11. The diaper 10 further may include a front waist region 13, a rear waist region 14, and a crotch region 15 positioned between the front and rear waist regions 13, 14.

### <Waist panel>

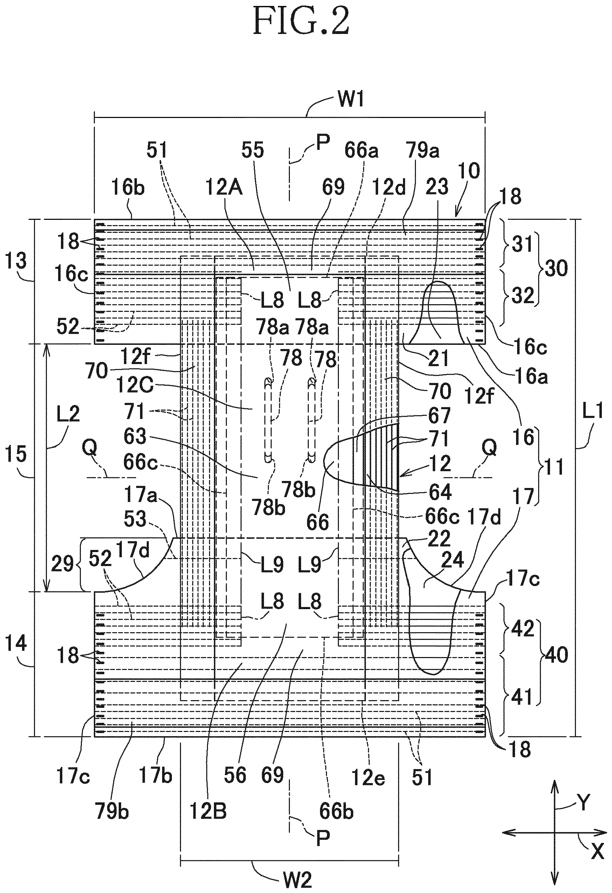

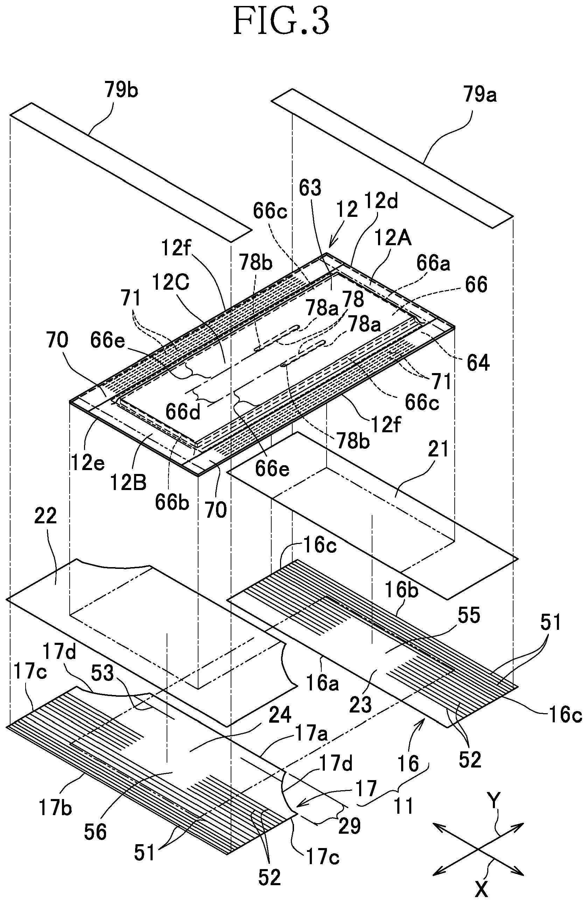

The waist panel 11 may include a front waist panel 16 forming the front waist region 12 and a rear waist panel 17 forming the rear waist region 14. The front waist panel 16, as shown in Fig. 3, is substantially rectangular shaped, and has a proximal end edge 16a extending in the lateral direction, intersecting with a front end edge 12A of the liquid absorbent structure 12, a distal end edge 16b extending in the lateral direction, spaced apart to be opposite to the proximal end edge 16a in the vertical direction, and both lateral side edges 16c extending in the vertical direction between the proximal end edge 16a and the distal end edge 16b. The rear waist panel 17, as shown in Fig. 2 and Fig. 3, is generally rectangular-shaped, and has a proximal end edge 17a extending in the lateral direction, parallel to a rear end edge 12B of the liquid absorbent structure 12, a distal end edge 17b extending in the lateral direction, spaced apart to be opposite to the proximal end edge 17a in the vertical direction, both lateral side edges 17c extending in the vertical direction between the proximal end edge 17a and the distal end edge 17b, and both corner edges 17d connecting the lateral side edges 17c and the proximal end edge 17a, and extending to be curved. Here, the "front waist region 13" in the present embodiment refers to a region defined by the front waist panel 16, the "rear waist region 14" refers to a region defined by the rear waist panel 17 (a region having a buttock cover part 29 that will be described later), and the "crotch region 15" refers to a region defined by the liquid absorbent structure 12, positioned between the front waist region 13 and the rear waist region 14.

Both lateral side edges 16c of the front waist panel 16 and both lateral side edges 17c of the rear waist panel 17, opposing each other in a frontward-rearward direction in the worn state, are overlapped with each other, and are joined along respective side seams 18 arranged at intervals in the vertical direction, and a waist opening 19 and a pair of leg openings 20 are defined. For the side seams 18, sheets overlapping each other are fused by an embossing/debossing process by thermal embossing/debossing or ultrasonic processing. The rear waist region 14 has a dimension in the vertical direction larger than that of the front waist region 13, and has the buttock cover part 29 extending downward from the side seams 18. The buttock cover part 29 has a generally trapezoidal shape with a width thereof narrowing gradually from the rear waist region 14 side toward the crotch region 15 side, and has a size that may cover the buttocks of the wearer. The rear waist panel 17 may be an oblong rectangular-shaped panel similar to the front waist panel 16, not having the buttock cover part 29.

### <Front waist panel>

Referring to Fig. 2, the front and rear waist panels 16, 17 may respectively include inner-layer sheets 21, 22 positioned on the skin facing surface, outer-layer sheets 23, 24 positioned on the skin non-facing surface, and waist elastic members 51, 52, and 53 formed of a plurality of string or strand-like elastic materials extending in the lateral direction, and contractively attached in a stretched state with hot-melt adhesive between the inner and outer-layer sheets 21, 22 and 23, 24.

The inner and outer-layer sheets 21, 22 and 23, 24 may be secured with the hot-melt adhesive applied to any inner surface thereof, or both sheets may be secured with hot-melt adhesive applied to a periphery of each of the waist elastic members.

### <Elastic areas>

Front and rear waist elastic areas 30, 40 stretchable at least in the lateral direction by the waist elastic members 51, 52 are defined in the front and rear waist regions 13, 14. The front and rear waist elastic areas 30, 40 may include upper elastic areas 31, 41 extending in the lateral direction on a waist-opening edge (distal end edges 16b, 17b) side and lower elastic areas 32, 42 extending in the lateral direction on the inner end edges 16a, 17a side. A plurality of first waist elastic members 51 and second waist elastic members 52 extending in the lateral direction is arranged in each of the upper elastic areas 31, 41 and the lower elastic areas 32, 42. The lower elastic area 32 in the front waist region 13 and the lower elastic area 42 in the rear waist region 14 are defined by a pair of elastic areas spaced apart to be opposite to each other in the lateral direction, and inelastic areas 55, 56, in which no waist elastic members are arranged or where elastic members are arranged in a practically inelastic state, are positioned in a middle part in the lateral direction of the front and rear waist regions 13, 14 defined between the pair of elastic areas. In such manner, by the inelastic areas 55, 56 being defined in the front and rear waist regions 13, 14, the elastic areas 30, 40 having an elastic force exerted by the first and second waist elastic members 52 positioned in the upper and lower elastic areas 32, 42 do not overlap with the absorbent body 66 in a plan view.

Moreover, a third waist elastic member 53 extending in the lateral direction is disposed between both corner edges 17d (an area of the buttock covering part 29) of the rear waist region 14. Although the third waist elastic member 53 is formed of a single elastic material in the illustration in the diagram, a plurality of elastic materials may be disposed. In that case, the plurality of elastic materials may be disposed evenly all over the buttock covering part 29. In the worn state of the diaper 10, gathers 58 are formed by a contractile force of the elastic members 51, 52, 53 (refer to Fig. 1).

Here, the front waist elastic area 30 refers to an area of the front waist region 13, or in other words, an area between the both lateral side edges 16c of the front waist region 13, and the rear waist elastic area 40 refers to an area of the rear waist region 14, or in other words, a portion between both lateral side edges 17c and a portion between the both corner edges 17d (the buttock covering part 29) put together.

The first waist elastic member 51 may include a string- or strand-like elastic material of a fineness in the range of 310 dtex to 940 dtex, elongated 2.0 to 2.8 times, preferably, 2.4 to 2.7 times, from a contracted or relaxed state. The second waist elastic members 52 may include a string- or strand-like elastic material of a fineness in the range of 310 dtex to 940 dtex, elongated 2.6 to 3.6 times, preferably, 2.9 to 3.4 times from a contracted or relaxed state. The third waist elastic members 53 may include a string- or strand-like elastic material of a fineness in the range of 310 dtex to 940 dtex, elongated 1.5 to 2.5 times, preferably, 1.7 to 2.1 times from a contracted or relaxed state.

Referring to Fig. 2, a dimension L1 in the vertical direction of the diaper 10 is in the range of 470 mm to 560 mm, a dimension (a dimension between the pair of seams 18) W1 in the lateral direction of the diaper 10 is in the range of 340 mm to 420 mm, a dimension L2 in the vertical direction of the crotch region 15 is in the range of 210 mm to 290 mm, and a dimension W2 in the lateral direction of the liquid absorbent structure 12 is in the range of 170 mm to 210 mm. Here, the dimensions L1, L2, W1, W2, W3, W4, W5, W6, and W7 are dimensions of the diaper 10 in the unfolded state, when measured in a state of being stretched in the vertical direction and the lateral direction to an extent such that the gathers by contraction action of the elastic members are not formed.

Referring to Fig. 2, Fig. 3, and Fig. 4, the liquid absorbent structure 12 has a generally rectangular shape, and has a front end part 12A attached to the skin facing surface of the front waist panel 16, a rear end part 12B attached to the skin facing surface of the rear waist panel 17, and an intermediate part 12C forming the crotch region 15 extending in the vertical direction between the front and rear end parts 12A, 12B. When hot-melt adhesive is applied to an entire inner surface of any one of the liquid absorbent structure 12 and the waist panel 11, the flexibility thereof is lost. Thus, preferably, the liquid absorbent structure 12 and the waist panel 11 are connected partially, for example, by applying the hot-melt adhesive to the two spirally or in dots, and/or by applying it in lines.

Referring to Fig. 2 and Fig. 3, the liquid absorbent structure 12 may include a liquid permeable body-side liner 63, positioned on the skin facing surface side, a water repellent covering sheet (outer surface sheet) 64, positioned on the skin non-facing surface side, and a liquid absorbent body 66, in the form of a pad interposed between the body-side liner 63 and the covering sheet 64. The body-side liner 63 may be formed of spun-bonded fibrous nonwoven fabrics and SMS fibrous nonwoven fabrics subjected to a hydrophilic treatment, having a mass in the range of 15 g/m² to 30 g/m². The covering sheet 64 may be formed of water repellent SMS fibrous nonwoven fabrics and spun-bonded nonwoven fabrics having a mass in the range of 8 g/m² to 15 g/m². A leakage barrier sheet 67 made of a plastic film having an air-through property and a mass in the range of 15 g/m² to 22 g/m² is disposed between the covering sheet 64 and the absorbent body 66. The liquid absorbent structure 12 further has end flaps 69 formed by the covering sheet 64 and the body-side liner 63 extending outward in the vertical direction of the front and rear end edges 66a, 66b of the absorbent body 66 and elastic side flaps 70 formed by the body-side liner 63 extending outward in the lateral direction of both lateral side edges of liquid absorbent body 66c, the covering sheet 64, and the leakage barrier sheet 67. When hot-melt adhesive is applied to an entire inner surface of any one of a core wrapping sheet wrapping an absorbent core and the body-side liner 63, the core wrapping sheet and the leakage barrier sheet 67, and the leakage barrier sheet 67 and the covering sheet 64, the flexibility thereof is lost, and thus, preferably, they are connected partially, for example, preferably, by applying the hot-melt adhesive spirally or in dots, and/or by applying it in lines.

At both lateral sides of the liquid absorbent structure 12, a part of the covering sheet 64 is folded inward and secured, and a leg elastic member 71 formed of a plurality of strings or strands-like elastic material of a fineness in the range of 470 dtex to 780 dtex, elongated 2.0 to 2.8 times from a contracted or relaxed state, extending in the vertical direction, is disposed between the folded part and the covering sheet 64 opposite to the folded part. Both lateral sides (side flaps 70) of the liquid absorbent structure 12 may include leg elastic areas 72 on which a stretching force of the leg elastic members 71 act, and leg inelastic areas 73 in which the leg elastic members 71 are not disposed, positioned at an inner side in the lateral direction of the leg elastic area 72. The plurality of leg elastic members 71 is arranged such that an interval in the lateral direction is the same.

A portion of both lateral sides of the covering sheet 64 is folded inward and secured, and the leg elastic members 71 formed of a plurality of strings- or strands-like elastic material of a fineness in the range of 310 dtex to 940 dtex, elongated 2.2 to 2.7 times from a contracted or relaxed state, extending in the vertical direction, is arranged between the folded part and the covering sheet 64 opposite to the folded part.

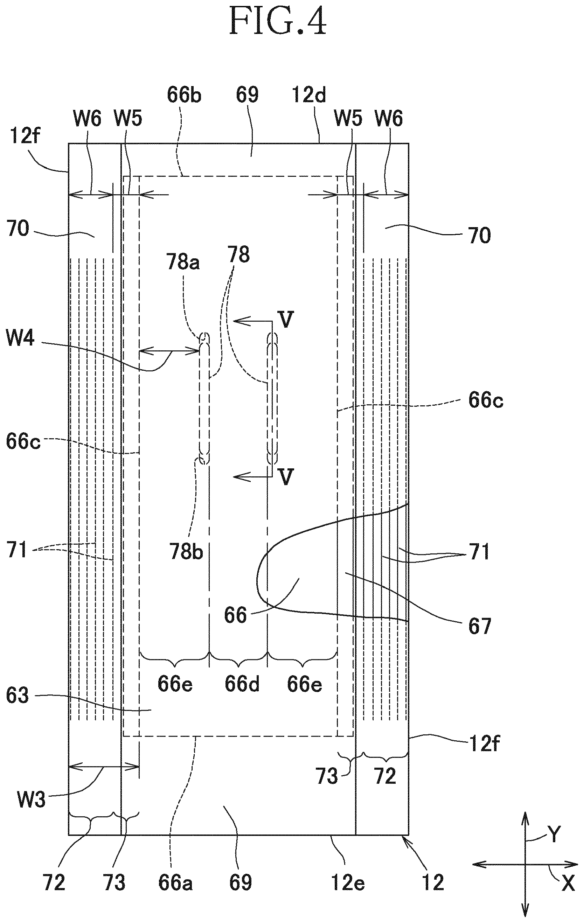

The absorbent body 66 may include an absorbent core formed of absorbent materials such as fluff wood pulp and super absorbent polymer particles, and a core wrapping sheet that is liquid impermeable and formed of tissue paper having a mass in the range of 13 g/m² to 20 g/m², wrapping the entire absorbent core. As illustrated in Fig. 2 and Fig. 4, the absorbent body 66 has a generally rectangular shape defined by a peripheral edge of the front and rear end edges 66a, 66b positioned in the front and rear waist regions 13, 14, and both lateral side edges 66c extending in the vertical direction between the front and rear end edges 66a, 66b. The liquid absorbent body 66 further may include a middle part 66d positioned at the middle in the lateral direction, and both lateral sides 66e positioned at both sides in the lateral direction. The liquid absorbent body 66 may include deformation guiding portions 78 extending in the vertical direction, positioned inboard of the front and rear end edges 66a, 66b and both lateral side edges 66c, and positioned in both lateral sides 66e.

The deformation guiding portions 78 are each formed into the shape of a concave groove, concave toward the skin non-facing surface from the skin facing surface, or a slot, in which the absorbent core is not present. The deformation guiding portions 78 are disposed to be spaced apart from each other at a predetermined dimension in the lateral direction in the crotch region 15.

The deformation guiding portions 78 may be a concave groove having a bottom, formed by pressurizing or removing a part of the absorbent body 66. In the case in which the deformation guiding portions 78 are formed by pressurizing, the deformation guiding portions 78 become an area having a high density and high rigidity compared to other areas of the absorbent body 66. Moreover, there may be two or more than two deformation guiding portions 78, and a part or a whole of the deformation guiding portions 78 may be curved and not extended linearly in the vertical direction as a whole.

The deformation guiding portions 78 have both guiding ends 78a, 78b in the vertical direction. Both guiding ends 78a, 78b are positioned in the crotch region 15, and the deformation guiding portions 78 do not overlap with the front and rear waist regions 13, 14 in a plan view.

The guiding both ends 78a, 78b may be inclined such that a dimension in a direction of thickness become gradually smaller from the skin facing surface side toward the skin non-facing surface side, in a direction of thickness Z orthogonal to the lateral direction and the vertical direction. However, the dimension in the direction of thickness of both guiding ends 78a, 78b may not be changed, so long as a technical effect of the present invention is achieved.

Elongate covering sheets 79a, 79b for the front and rear end edges 12d, 12e of the liquid absorbent structure 12 are attached to the front and rear end edges 12d, 12e. The elongate covering sheets 79a, 79b may be formed of materials such as fibrous nonwoven fabrics and plastic sheets having a mass in the range of 13 g/m² to 20 g/m², and are secured with hot-melt adhesive (not shown) to the skin facing surface of the front and rear waist panels 16, 17 while covering the front and rear end edges 12d, 12e of the liquid absorbent structure 12. The liquid absorbent structure front and rear end edges 12d, 12e, being covered by the elongate covering sheets 79a, 79b, do not come into contact with the skin of the wearer.

Moreover, in the rear waist elastic area 40, the lower elastic areas 42 and the elastic side flaps 70 are joined while overlapping with each other in a plan view. In such a manner, the lower elastic area 42 and the elastic side flaps 70 intersect with each other, and a virtual annular elastic belt is formed about the legs of the wearer by a combination of the lower elastic area 42 and the leg elastic area acting as a stretching force of the leg elastic members 71. This makes it possible to prevent the leakage of body exudates from sites of the leg openings surrounding the thighs.

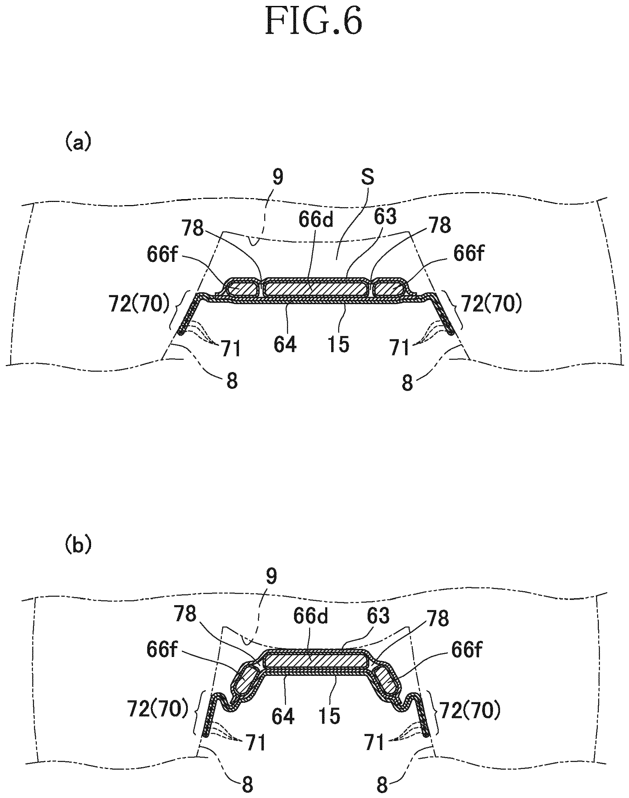

Referring to Fig. 6 (a), at the time of putting on the diaper 10, both legs of the wearer are inserted into the leg openings 20, and in a state of the diaper 10 pulled adjacent to the crotch 9 of the wearer by holding both lateral sides of the diaper by the wearer or a caretaker, the absorbent body 66 is retained in an almost flat state, and the elastic side flaps 70 are brought into contact with an inner side of the thighs 8. The absorbent body 66 is positioned away from the crotch 9 in a flat state at the lower side of the crotch 9, and a space S is formed between the absorbent body 66 and the crotch 9.

Referring to Fig. 6 (b), from the abovementioned state, when the wearer has pulled the diaper 10 further up, the side flaps 70 are held in position in contact with the inner side of the thigh portion 8 because of a frictional resistance of the skin of the wearer and the elastic side flaps 70. The absorbent body 66, under the effect of the deformation guiding portions 78, is deformed to be concave downward with respect to the deformation guiding portions 78 (such that the middle part 66d is positioned at an upper side, and side portions 66f of the absorbent body 66 are positioned at an outer side in the lateral direction of the deformation guiding portions 78). While the elastic side flaps 70 are held in the contact position at the time of putting on the diaper 10, the absorbent body 66 comes closer to the crotch 9 by being deformed to be concave, and the space S becomes smaller gradually, and the absorbent body 66 may be brought into close contact with the crotch.

In such a manner, the absorbent body 66 is maintained such that the elastic side flaps 70 at the lower part of the absorbent body 66 are stretched upon being brought into contact with the thigh portion 8, the absorbent body 66 may be brought into close contact with the crotch 9 of the wearer, thereby letting the body fluids be absorbed adequately as well as to prevent effectively the side leakage of the body fluids from between the side flaps 70 and the thigh portion 8. Moreover, the space S not being formed between the absorbent body 66 and the crotch 9, the fittability of the crotch region 15 is improved and a good wearing feeling may be provided.

A width dimension of the crotch region 15 and a width dimension of the absorbent body 66 vary according to the size of the diaper 10, and in the diaper 10 of the present embodiment, by a dimension W3 in the lateral direction of each of the side flaps 70 being larger than a dimension W4 in the lateral direction from both lateral edges 66c of the absorbent body 66 to each of the deformation guiding portions 78, the elastic side flaps 70 have a relatively broad width, and as compared to a case of the elastic side flaps 70 having a relatively narrow width, a frictional resistance force thereof is strong, and can be said to be held on at a position when brought into contact with the thighs 8 of the wearer. Moreover, when an attempt is made to pull the diaper 10 up, in the case in which the deformation guiding portions 78 are positioned at the middle part side of the absorbent body 66, or in other words, in the case in which both lateral sides 66e of the absorbent body 66 are relatively wide, a force that makes the elastic side flaps 70 to be held on at the position of contact of the thighs 8 by the elasticity thereof acts on the entire absorbent body 66, and the force that pulls the diaper 10 up may be hard to act adequately on the absorbent body 66.

In the present embodiment, both lateral sides 66e of the absorbent body 66 being relatively narrow in width, the force that makes the absorbent body 66 be held on by the elastic side flaps 70 in the deformation guiding portions 78 is divided, and the middle part 66d is easy to be pulled up toward the crotch 9 of the wearer. Moreover, a width dimension of the middle part 66d of the absorbent body 66 being relatively large, the body fluids may be absorbed and contained in the middle part 66d that has come in close contact with the crotch 9. To achieve such an effect, preferably, the dimension W3 in the lateral direction of the elastic side flaps 70 is in the range of 25 mm to 45 mm, and the dimension W4 in the lateral direction from both lateral edges 66c of the absorbent body 66 to each of lateral edges of the deformation guiding portions 78 is in the range of 20 mm to 40 mm. However, even when W3 and W4 are set in the abovementioned ranges respectively, it is preferable that the dimension W3 in the lateral direction of the elastic side flaps 70 is larger than the dimension W4 in the lateral direction from both lateral edges 66c of the absorbent body 66 to each of the lateral edges of the deformation guiding portions 78, and it is preferable that a dimension W6 in the lateral direction of the leg elastic area 72 is larger than the dimension W4 in the lateral direction from both lateral edges 66c of the absorbent body 66 to each of the lateral edges of the deformation guiding portions 78.

The deformation guiding portions 78, in addition to functioning as fold starting lines for deforming the absorbent body 66 and bringing the middle part 66d thereof closer to the wearer's body, also has a function as a guide groove for diffusing the body exudates absorbed, over the entire absorbent body 66. Moreover, the deformation guiding portions 78 are positioned only in the crotch region 15 opposite to the crotch 9 of the wearer. The deformation guiding portions 78 not being positioned in the front and rear waist regions 13, 14, the absorbent body 66 is not deformed in the front and rear waist regions 13, 14, and the outside appearance of the diaper is not disfigured because of the deformation.

In the worn state of the diaper as shown in Fig. 1, the deformation guiding portions 78 have the guiding ends 78a, 78b at both ends in the vertical direction. The deformation guiding portions 78 are provided on the absorbent body 66 in the vertical direction such that the guiding end 78a, that is one of the guiding ends 78a, 78b, is positioned on the front waist region 13 side and the other guiding end 78b is positioned on the rear waist region 14 side. This enables, at the front waist region 13 side of the crotch region 15, the prevention of body exudates such as urine from leaking by deformation of the absorbent body 66 when it is brought into close contact with the skin of the wearer, and, at the rear waist region 14 side of the crotch region 15, it prevents the deformation of the absorbent body 66, and enables wide coverage of the buttocks.

Referring to Fig. 2, the absorbent body 66, has the middle part 66d with the absorbent core continuous in the vertical direction, between both lateral sides 66c, and the rear waist region 14 has the pair of lower elastic areas 42 spaced apart to be opposite in the lateral direction, and the inelastic area 56 positioned between the lower elastic areas 42, and the deformation guiding portions 78 are positioned between a pair of virtual lines L9 extending along a boundary line L8 of the lower elastic areas 42 and the inelastic area 56 toward the crotch region 15. By the deformation guiding portions 78 having such an arrangement, and having the pair of lower elastic areas 42 and the inelastic area 56 positioned between the elastic areas (lower elastic areas 42), when the wearer or the caretaker fits the rear waist region 14 to the buttocks by pulling the diaper 10 up by holding the middle part in the lateral direction of the rear waist region 14, a force pulling up by a stretching action of the lower elastic areas 42, without being dispersed in a direction about the waist, acts directly on the middle part 66d positioned between the deformation guiding portions 78 positioned at the lower side, and the absorbent body 66 is deformed, and the middle part 66d is pulled up, thereby enabling a fit to the buttocks of the wearer.

The elastic side flaps 70 have the plurality of leg elastic members 71 extending in the vertical direction, and base sheets having the leg elastic members 71 attached thereto. The base sheets of the elastic side flaps 70 are formed of the covering sheet 64 positioned on the skin non-facing surface side of the liquid absorbent structure 12. In the case in which the base sheet of the side flap 70 is formed of a sheet other than the covering sheet 64 of the liquid absorbent structure 12, the body fluids may be leaked from a side where the sheets are joined, but the sheets formed integrally enables prevention of body exudates from leaking from the side.

In the worn state, while the leg inelastic area 73 of the side flap 70 is raised toward the body of the wearer, the leg elastic area 72 fits to the inner side thereof, along the thighs . For exerting the abovementioned effect, preferably, a dimension W5 in the lateral direction of the leg inelastic area 72 is in the range of 5 mm to 15 mm, and preferably, the dimension W6 in the lateral direction of the leg elastic area 72 is in the range of 15 mm to 35 mm. Moreover, preferably, a ratio of the dimension W5 in the lateral direction of the leg inelastic area 73 to the dimension W3 in the lateral direction of the side flap 70 (W5 / W3) is in the range of 11% to 60%, and a ratio (proportion) of the dimension W6 in the lateral direction of the leg elastic area 72 to the dimension W3 in the lateral direction of the side flap 70 (W6 / W3) is in the range of 33% to 95%.

### <First modified embodiment>

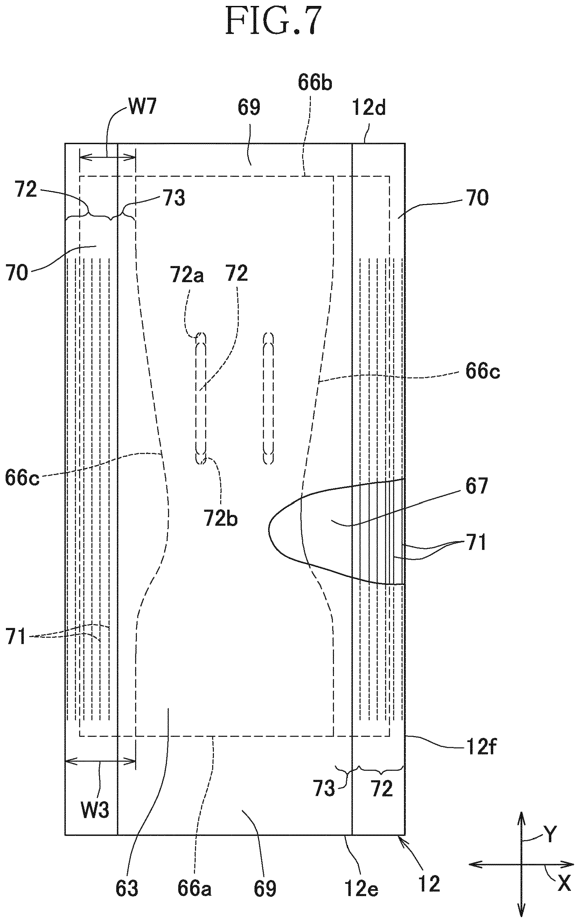

Fig. 7 is a plan view similar to Fig. 4, illustrating an example of a first modified embodiment of the diaper 10. For the leakage barrier sheet 67 in this diaper 10, a dimension W7 in the lateral direction of the leakage barrier sheet 67 positioned inside the side flap 70 with respect to the dimension W3 in the lateral direction of the side flap 70 is 50% or more. Thus, the absorbent body 66 and the side flap 70 being connected by the leakage barrier sheet 67, when the side flaps 70 are brought into close contact with the skin of the wearer by the elasticity of the side flap 70, both lateral sides 66e of the absorbent-body may be brought into close contact with the skin of the wearer, and thus, to prevent the body exudates from leaking from the diaper 10. Moreover, preferably, the dimension W7 in the lateral direction of the leakage barrier sheet 67 positioned inside the side flap 70 is in the range of 20 mm to 60 mm.

Moreover, the absorbent body 66 may be formed to have the front and rear end edges 66a, 66b positioned in the front and rear waist regions 13, 14, and both lateral edges 66c having a concave shape and extending in the vertical direction between the front and rear end edges 66a, 66b. Both lateral edges 66c being concave-shaped, the dimension in the lateral direction of the absorbent body 66 may be made small, and accordingly, the legs of the wearer are prevented from being brought into contact with both lateral edges 66c, and the degradation in comfort of wearing may be prevented.

### <Second modified embodiment>

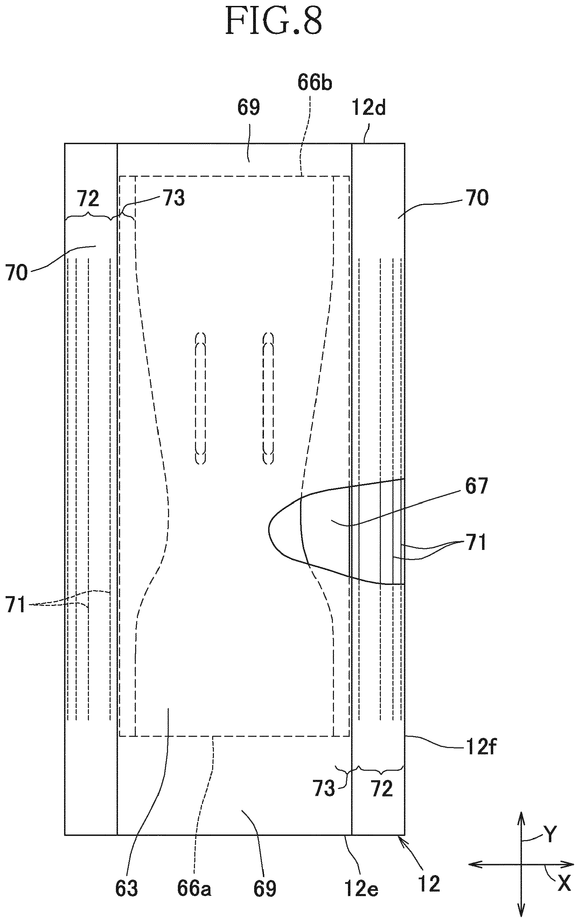

Fig. 8 is a plan view similar to Fig. 4, illustrating an example of a second modified embodiment of the diaper 10. An interval of the leg elastic members 71 in this diaper 10 becomes narrower toward both lateral edges 12f of the liquid absorbent structure. Thus, the closer to the liquid absorbent structure both lateral edges 12f, the firmer is the close contact with the skin of the wearer, and the body exudates may be prevented from leaking from the diaper 10.

In the abovementioned embodiment, an arrangement in which the diaper 10 includes the three panels which are, the liquid absorbent structure 12, the front waist panel 16, and the rear waist panel 17, was described. However, the present invention is not restricted to such arrangement, and the arrangement may be such that in the diaper 10, the liquid absorbent structure is attached to a skin facing surface of integrated inner and outer-layer sheets extending from the front and rear waist regions 13, 14 toward the crotch region 15.

Moreover, in the abovementioned embodiment, the diaper 10 has the deformation guiding portion 78 provided in the vertical direction such that one guiding end part 78a is positioned on the side of the front waist region 13 and is positioned at the upper side of the other guiding end 78b which is positioned on the side of the rear waist region 14. However, the present invention is not restricted to such arrangement, and the deformation guiding part 78 may not be biased in the vertical direction.

Furthermore, the abovementioned embodiment and modified embodiments can be adopted upon isolating or combining with each other.

For each of the components forming the diaper 10, unless described in particular, various known materials generally used in this type of field may be used without restrictions. Moreover, the terms such as 'the first' and 'the second' used in the description of the present invention are used simply for distinguishing similar components and positions.

### [Reference Signs List]

- 10: disposable wearable article (disposable diaper)
- 12: liquid absorbent structure
- 12f: both lateral edges
- 13: front waist region
- 14: rear waist region
- 15: crotch region
- 40: elastic area
- 56: inelastic area
- 64: covering sheet (outer surface sheet)
- 66: absorbent body
- 66e: absorbent-body lateral sides
- 67: leakage barrier sheet
- 70: side flap
- 71: leg elastic member
- 78: deformation guiding portion
- 78a: one guiding end
- 78b: other guiding end part
- L8: boundary line
- L9: virtual line
- W3: dimension in lateral direction of side flap
- W4: dimension in lateral direction from both lateral edges to deformation guiding portion
- X: lateral direction
- Y: vertical direction

## Claims

1. A disposable wearable article (10) having a vertical direction and a lateral direction, comprising:
a front waist region (13);
a rear waist region (14);
a crotch region (15) positioned between the front and rear waist regions; and
an absorbent body (66) positioned at least in the crotch region (15), wherein
the absorbent body has both lateral sides (66e) opposing each other in the lateral direction in the crotch region, and a deformation guiding portion (78) extending in the vertical direction in each of the lateral sides (66e),
elastic side flaps (70) extending in the vertical direction are positioned at outer sides in the lateral direction of both of the lateral sides (66e), and
a dimension (W3) in the lateral direction of each of the elastic side flaps (70) is larger than a dimension (W4) in the lateral direction from each lateral edge (66c) of the absorbent body to a lateral outside edge of each of the deformation guiding portions (78).

2. The wearable article according to claim 1, wherein the deformation guide portions (78) are positioned only in the crotch region (15).

3. The wearable article according to claim 1 or 2, wherein each of the deformation guiding portions (78) has guiding ends at both ends in the vertical direction, and
in a worn state of the article, the deformation guiding portions (78) are provided in the vertical direction such that one guiding end (78a) is positioned on a side of the front waist region (13) and is positioned above the other guiding end (78b) which is positioned on a side of the rear waist region (14).

4. The wearable article according to any one of claims 1 through 3, wherein
the rear waist region (14) has a pair of elastic areas (42) spaced apart to be opposite in the lateral direction, and an inelastic area (56) positioned between the elastic areas, and
the deformation guiding portions (78) are positioned between a pair of virtual lines (L9) extending along a boundary line (L8) of the elastic area and the inelastic area toward the crotch region (15).

5. The wearable article according to any one of claims 1 through 4, further comprising:
a liquid absorbent structure (12) including the absorbent body (66) and an outer surface sheet (64) positioned on the skin non-facing surface side of the absorbent body, wherein
each of the elastic side flaps (70) has a plurality of leg elastic members (71) extending in the vertical direction, and a base sheet having the leg elastic members attached thereto, and
the base sheet and the outer surface sheet (64) are formed integrally.

6. The wearable article according to claim 5, wherein
the liquid absorbent structure (12) further includes leakage barrier sheets (67) that are liquid impermeable, interposed between the absorbent body (66) and the outer surface sheet (64), and
the leakage barrier sheets extend from both the lateral edges (66c) of the absorbent body to further outer side in the lateral direction, and
a dimension in the lateral direction of the leakage barrier sheets (67) positioned in each of the elastic side flaps (70) is not less than 50% of the dimension in the lateral direction of each of the elastic side flaps.

7. The wearable article according to claim 5 or 6, wherein an interval of the leg elastic members (71) becomes narrower gradually toward both lateral side edges (12f) of the liquid absorbent structure (12).

## Patentansprüche

1. Tragbarer Einwegartikel (10), der eine vertikale Richtung und eine laterale Richtung aufweist, Folgendes umfasst:
einen vorderen Taillenbereich (13);
einen hinteren Taillenbereich (14);
einen Schrittbereich (15), der zwischen dem vorderen und hinteren Taillenbereich positioniert ist; und
einen Saugkörper (66), der zumindest in dem Schrittbereich (15) positioniert ist, wobei
der Saugkörper Folgendes aufweist: beide lateralen Seiten (66e), die einander in der lateralen Richtung in dem Schrittbereich gegenüberliegen, und einen Verformungsführungsteil (78), der sich in der vertikalen Richtung in jeder der lateralen Seiten (66e) erstreckt,
elastische Seitenklappen (70), die sich in der vertikalen Richtung erstrecken, an äußeren Seiten in der lateralen Richtung von beiden der lateralen Seiten (66e) positioniert sind und
eine Abmessung (W3) in der lateralen Richtung von jeder der elastischen Seitenklappen (70) größer ist als eine Abmessung (W4) in der lateralen Richtung von jedem lateralen Rand (66c) des Saugkörpers zu einem lateralen äußeren Rand von jedem der Verformungsführungsteile (78).

2. Tragbarer Artikel nach Anspruch 1, wobei die Verformungsführungsteile (78) nur in dem Schrittbereich (15) positioniert sind.

3. Tragbarer Artikel nach Anspruch 1 oder 2, wobei jeder der Verformungsführungsteile (78) Führungsenden an beiden Enden in der vertikalen Richtung aufweist und,
in einem getragenen Zustand des Artikels, die Verformungsführungsteile (78) in der vertikalen Richtung derart bereitgestellt sind, dass ein Führungsende (78a) an einer Seite des vorderen Taillenbereichs (13) positioniert ist und über dem anderen Führungsende (78b) positioniert ist, das an einer Seite des hinteren Taillenbereichs (14) positioniert ist.

4. Tragbarer Artikel nach einem der Ansprüche 1 bis 3, wobei
der hintere Taillenbereich (14) Folgendes aufweist: ein Paar von elastischen Flächen (42), die beabstandet sind, um in der lateralen Richtung gegenüberzuliegen, und eine unelastische Fläche (56), die zwischen den elastischen Flächen positioniert ist, und
die Verformungsführungsteile (78) zwischen einem Paar von virtuellen Linien (L9) positioniert sind, die sich entlang einer Begrenzungslinie (L8) der elastischen Fläche und der unelastischen Fläche in Richtung des Schrittbereichs (15) erstrecken.

5. Tragbarer Artikel nach einem der Ansprüche 1 bis 4, der weiter Folgendes umfasst:
eine flüssigkeitsabsorbierende Struktur (12), die den Saugkörper (66) und eine äußere Oberflächenlage (64) einschließt, die auf der nicht der Haut zugewandten Oberflächenseite des Saugkörpers positioniert ist, wobei
jede der elastischen Seitenklappen (70) Folgendes aufweist: eine Vielzahl von elastischen Beinelementen (71), die sich in der vertikalen Richtung erstrecken, und eine Grundlage, die die elastischen Beinelemente daran angebracht aufweist, und
die Grundlage und die äußere Oberflächenlage (64) einteilig ausgebildet sind.

6. Tragbarer Artikel nach Anspruch 5, wobei
die flüssigkeitsabsorbierende Struktur (12) weiter Auslaufbarrierelagen (67) einschließt, die flüssigkeitsundurchlässig, zwischen den Saugkörper (66) und die äußere Oberflächenlage (64) eingefügt sind, und
sich die Auslaufbarrierelagen von beiden der lateralen Ränder (66c) des Saugkörpers zur weiter äußeren Seite in der lateralen Richtung erstrecken und
eine Abmessung in der lateralen Richtung der Auslaufbarrierelagen (67), die in jeder der elastischen Seitenklappen (70) positioniert sind, nicht weniger als 50 % der Abmessung in der lateralen Richtung von jeder der elastischen Seitenklappen beträgt.

7. Tragbarer Artikel nach Anspruch 5 oder 6, wobei ein Abstand der elastischen Beinelemente (71) in Richtung der beiden lateralen Seitenränder (12f) der flüssigkeitsabsorbierenden Struktur (12) allmählich schmaler wird.

## Revendications

1. Article vestimentaire jetable (10) ayant une direction verticale et une direction latérale, comportant :
une région avant au niveau de la taille (13) ;
une région arrière au niveau de la taille (14) ;
une région au niveau de l'entrejambe (15) positionnée entre les régions avant et arrière au niveau de la taille ; et
un corps absorbant (66) positionné au moins dans la région au niveau de l'entrejambe (15), dans lequel
le corps absorbant a les deux côtés latéraux (66e) orientés de manière opposée l'un par rapport à l'autre dans la direction latérale dans la région au niveau de l'entrejambe, et une partie de guidage de déformation (78) s'étendant dans la direction verticale dans chacun des côtés latéraux (66e),
des rabats latéraux élastiques (70) s'étendant dans la direction verticale sont positionnés au niveau des côtés extérieurs dans la direction latérale des deux côtés latéraux (66e), et
une dimension (W3) dans la direction latérale de chacun des rabats latéraux élastiques (70) est supérieure à une dimension (W4) dans la direction latérale depuis chaque bord latéral (66c) du corps absorbant jusqu'à un bord extérieur latéral de chacune des parties de guidage de déformation (78).

2. Article vestimentaire selon la revendication 1, dans lequel les parties de guidage de déformation (78) sont positionnées uniquement dans la région au niveau de l'entrejambe (15).

3. Article vestimentaire selon la revendication 1 ou la revendication 2, dans lequel chacune des parties de guidage de déformation (78) a des extrémités de guidage au niveau des deux extrémités dans la direction verticale, et
dans un état porté de l'article, les parties de guidage de déformation (78) sont mises en œuvre dans la direction verticale de telle sorte qu'une extrémité de guidage (78a) est positionnée sur un côté de la région avant au niveau de la taille (13) et est positionnée au-dessus de l'autre extrémité de guidage (78b) qui est positionnée sur un côté de la région arrière au niveau de la taille (14).

4. Article vestimentaire selon l'une quelconque des revendications 1 à 3, dans lequel
la région arrière au niveau de la taille (14) a une paire de zones élastiques (42) espacées l'une de l'autre pour être opposées dans la direction latérale, et une zone non élastique (56) positionnée entre les zones élastiques, et
les parties de guidage de déformation (78) sont positionnées entre une paire de lignes virtuelles (L9) s'étendant le long d'une ligne de délimitation (L8) de la zone élastique et de la zone non élastique vers la région au niveau de l'entrejambe (15).

5. Article vestimentaire selon l'une quelconque des revendications 1 à 4, comportant par ailleurs :
une structure absorbant les liquides (12) comprenant le corps absorbant (66) et une feuille de surface extérieure (64) positionnée sur la surface non orientée vers la peau du corps absorbant, dans lequel
chacun des rabats latéraux élastiques (70) a une pluralité d'éléments élastiques au niveau des jambes (71) s'étendant dans la direction verticale, et une feuille de base ayant les éléments élastiques au niveau des jambes attachés sur celle-ci, et
la feuille de base et la feuille de surface extérieure (64) sont formées d'une seule pièce.

6. Article vestimentaire selon la revendication 5, dans lequel
la structure absorbant les liquides (12) comprend par ailleurs des feuilles de barrière antifuite (67) qui sont imperméables aux liquides, intercalées entre le corps absorbant (66) et la feuille de surface extérieure (64), et
les feuilles de barrière antifuite s'étendent depuis les deux bords latéraux (66c) du corps absorbant jusqu'au côté le plus à l'extérieur dans la direction latérale, et
une dimension dans la direction latérale des feuilles de barrière antifuite (67) positionnées dans chacun des rabats latéraux élastiques (70) ne mesure pas moins de 50 % de la dimension dans la direction latérale de chacun des rabats latéraux élastiques.

7. Article vestimentaire selon la revendication 5 ou la revendication 6, dans lequel un intervalle des éléments élastiques au niveau des jambes (71) devient progressivement plus étroit vers les deux bords de côté latéraux (12f) de la structure absorbant les liquides (12).
